# EUROPÄISCHE PATENTSCHRIFT

(11) **EP 1 341 512 B1**
(45) Veröffentlichungstag und Bekanntmachung des Hinweises auf die Patenterteilung: **31.12.2008**
(21) Anmeldenummer: 01997290.0
(22) Anmeldetag: 12.11.2001
(51) Int. Cl.: A23G 4/06, A23L 1/226, A61K 31/075

(54) **RHINOLOGICA**
RHINOLOGICAL AGENTS
PRODUITS RHINOLOGIQUES

(30) Priorität: 24.11.2000 DE 10058459
(43) Veröffentlichungstag der Anmeldung: 10.09.2003
(73) Patentinhaber: Symrise GmbH & Co. KG, 37603 Holzminden (DE)
(72) Erfinder: SURBURG, Horst, 37603 Holzminden (DE); MACHINEK, Arnold, 37603 Holzminden (DE); LOGES, Hubert, 37671 Hoexter (DE)
(74) Vertreter: Eisenführ, Speiser & Partner
(86) Internationale Anmeldenummer: PCT/EP2001/013061
(87) Internationale Veröffentlichungsnummer: WO 2002/041861

(56) Entgegenhaltungen:
- EP-A- 0 262 388
- EP-A- 0 761 632
- JP-A- 11 152 235
- US-A- 3 993 604
- US-A- 4 130 509
- US-A- 4 275 080
- DATABASE CHEMABS [Online] CHEMICAL ABSTRACTS SERVICE, COLUMBUS, OHIO, US; OBATA, Y. ET AL: "Effect of synthesized cyclohexanol derivatives using L-menthol as a lead compound on the percutaneous absorption of ketoprofen" retrieved from STN Database accession no. 133:140073 XP002190755 & INT. J. PHARM. (2000), 198(2), 191-200 ,
- DATABASE CHEMABS [Online] CHEMICAL ABSTRACTS SERVICE, COLUMBUS, OHIO, US; CHASTRETTE, MAURICE ET AL: "Structure-minty odor relationships: suggestion of an interaction pattern" retrieved from STN Database accession no. 129:39036 XP002190756 & FLAVOUR FRAGRANCE J. (1998), 13(1), 5-18 ,
- DATABASE BEILSTEIN [Online] BEILSTEIN INSTITUTE FOR ORGANIC CHEMISTRY, FRANKFURT/MAIN, DE; Database accession no. brn3231261 XP002190757

## Beschreibung

Die Erfindung betrifft Rhinologica, die im Bereich des Mundes, des Rachens und der Atemwege ein frisches und befreiendes Gefühl hervorrufen und Zubereitungen, die diese Verbindungen enthalten.

1,8-Cineol (Eucalyptol) ist ein Stoff, der vor allem wegen seiner Eigenschaft, ein kühlend-frisches und damit befreiendes Empfinden in dem Bereich des Mundes, des Rachens und der Atemwege zu erzeugen, in großen Mengen als Rhinologicum in pharmazeutischen Präparaten, Mundpflegepräparaten, wie Zahnpasten und Mundwässern, und Süßwaren, wie Hustenbonbons und Kaugummis, eingesetzt wird. Neben diesem Effekt zeigt 1,8-Cineol (Eucalyptol) jedoch einen starken Eigengeschmack, der wegen seiner ausgeprägten medizinischen Note von vielen Verbrauchern als unangenehm empfunden wird.

Es besteht daher ein Bedürfnis an Stoffen, die ähnlich wie 1,8-Cineol (Eucalyptol) ein kühlend-frisches und damit befreiendes Gefühl als Rhinologicum im Bereich des Mundes, des Rachens und der Atemwege, speziell in der Nasenhöhle, und im Rachenraum erzeugen, aber nicht so einen starken und unangenehmen Eigengeschmack aufweisen.

Der Vorteil solcher Rhinologica liegt darin, dass sie universeller einsetzbar sind, d.h. in Zubereitungen mit einer Vielzahl von Aromen der unterschiedlichsten Geschmacksnoten verwendet werden können.

Niedere Alkylether des Isobornans, hier besonders der Methylisobornylether (Food Chem. Toxicol. 30 (Suppl.), 53S (1992), und des Bornans, z.B: Bomylmethylether und Bornylethylether, ( US 4.131.687) sind bereits seit längerem als Riech-, bzw. Aromastoffe mit frischen, krautigen, rosmarinartigen, bzw. eucalyptusartigen sensorischen Eigenschaften bekannt. Wegen ihres starken Eigengeschmackes sind die jedoch nicht als Ersatzprodukte für 1,8-Cineol (Eucalyptol) im oben beschriebenen Sinne verwendbar.

Es wurden Zubereitungen mit Frischewirkung, enthaltend Verbindungen der Formel 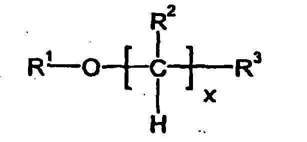 (Rhinologica),
worin
- x: den Wert 0 oder 1 annehmen kann,
- R¹: eine Alkylgruppe mit 1 bis 4 C-Atomen,
- R²: eine Methyl- oder Ethylgruppe und
- R³: ein monocyclisches Kohlenstoffsystem mit 5,6, 7 oder 8 Kohlenstoffatomen, das gegebenenfalls mit weiteren Alkylgruppen mit 1 bis 4 C-Atomen oder Alkenylgruppen mit 2 bis 4 C-Atomen substituiert sein kann, bedeutet,
in einer Konzentration von 0,001 bis 10 Gew.%
und ausgewählt aus der Gruppe, die im Anspruch 1 definiert ist gefunden.

Bei den erfindungsgemäß enthaltenen Rhinologica handelt es sich um acyclische Ether.

Alkyl mit 1 bis 4 Kohlenstoffatomen kann Methyl, Ethyl, Propyl, iso-Propyl, Butyl oder iso-Butyl sein.

Alkenyl mit 2 bis 4 Kohlenstoffatomen kann Vinyl, 2-Propenyl, Allyl oder 2-Buten-1-yl sein.

Der Rest R³ kann unsubstituiert oder beispielsweise mit 1 bis 3 Methylgruppen oder mit 1 iso-Propylgruppe oder mit 1 Methylgruppe und 1 iso-Propylgruppe oder mit 1 Ethylgruppe und 1 2-Propenylgruppe substituiert sein.

Die erfindungsgemäß enthaltenen Rhinologica zeigen eine dem 1,8-Cineol (Eucalyptol) vergleichbare Wirkung in Bezug auf ein frisches befreiendes Gefühl im Mund, Rachenraum und den Atemwegen, ohne dabei ein unangenehmes Geschmacksempfinden zu erzeugen.

Diese Rhinologica zeigen neben ihrer Wirkung, ein frisches befreiendes Gefühl im Mund, Rachenraum und den Atemwegen zu erzeugen, frische, etherische, minzige, kühlende, süßliche, und kachtige Geschmacksnoten und sind daher auch hervorragend als Aromastoffe geeignet.

Dass die erfindungsgemäß enthaltenen Rhinologica in Bezug auf ein frisches befreiendes Gefühl im Mund, Rachenraum und den Atemwegen eine dem 1,8-Cineol (Eucalyptol) vergleichbare Wirkung zeigen, war insofern überraschend und nicht voraussehbar, als die erfindungsgmäß enthaltenen Ether nicht wie 1,8-Cineol (Eucalyptol) eine cyclische, sondern vielmehr eine acyclische Struktur haben.

Bevorzugte Rhinologica im Rahmen der vorliegenden Erfindung sind Verbindungen der Formel 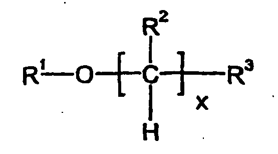 worin
- x: den Wert 0 oder 1 annehmen kann,
- R¹: eine Methyl- oder Ethylgruppe,
- R²: eine Methyl- oder Ethylgruppe und
- R³: ein monocyclisches Kohlenstoffsystem mit 6 oder 7 Kohlenstoffatomen, das gegebenenfalls mit weiteren Alkylgruppen mit 1 bis 3 C-Atomen und/oder Alkenylgruppen mit 3 C-Atomen substituiert sein können, bedeutet

Durch ihre vorzüglichen Eignung als angenehm schmeckende Aromastoffe mit der Wirkung, ein frisches befreiendes Gefühl im Mund, Rachenraum und den Atemwegen hervorzurufen, sind die erfindungsgemäß enthaltenen Rhinologica aus der Gruppe 1-Menthylmethylether, d-Menthylmethylether, dl-Menthylmethylether, Menthylethylether, Menthylpropylether, Menthylisobutylether, Isopulegylmethylether, 2-Isopropylcyclohexylmethylether, 2-Isopropylcyclohexylethylether, 3,3,5-Trimethylcyclohexylmethylether, 1-(3,3-Dimethylcyclohexyl)ethylethylether, 1-(3,3-Dimethylcyclohexyl)ethylpropylether, 1-(3,3-Dimethylcyclohexyl)ethylmethylether ausgewählt.

Die Wirkung, ein frisches befreiendes Gefühl im Mund, Rachenraum und den Atemwegen hervorzurufen, trifft bei den erfindungsgemäß enthaltenen Rhinologica auf alle isomeren Formen, d.h. Diastereomere und Enantiomere zu.

Folgende acyclischen Ether für die erfindangsgemäßen Rhinologica sind neu: Isopulegylmethylether, 1-(3,3-Dimethylcyclohexyl)-ethylethylether, 1-(3,3-Dimethylcyclohexyl)ethylpropylether und 1-(3,3-Dimethylcyclohexyl)ethylmethylether.

Die Herstellung der acyclischen Ether für die erfindungsgemäß enthaltenen Rhinologica ist an sich bekannt. Sie kann beispielsweise durch die Veretherung der entsprechenden Alkohole mit Alkylierungsmitteln wie Alkylhalogeniden, Alkyltosylaten, Alkylmesylaten oder Alkylhalogeniden in Gegenwart einer äquivalenten Menge einer basischen Verbindung erfolgen. Besonders vorteilhaft ist dabei die Veretherung nach dem Phasen-Transfer-Verfahren, die z.B. in Angew. Chem. 85, 868-869 (1973) beschrieben ist und wie folgt durchgeführt wird: Der zu verethernde Alkohol wird in einem unpolaren Lösungsmittel in Gegenwart eines Phasen-Transfer-Katalysators wie z.B. Tetrabutylammoniumiodid mit einem 2,5-fachen Überschuß an 50-prozentiger Natronlauge kräftig durchgerührt und mit einem 1,2-fachen Überschuß eines Alkylierungsmittels versetzt. Nach üblicher Aufarbeitung erhält man den entsprechenden Ether, der durch Destillation oder Flüssigchromatographie von nicht umgesetztem Alkohol abgetrennt wird.

Zur Erzielung von frischen, etherischen, minzigen, kühlenden, süßlichen und fruchtigen Geschmacksnoten in Verbindung mit einem frischen befreienden Gefühl im Mund, Rachenraum und den Atemwegen können die erfindungsgemäß enthaltenen Rhinologica in reiner Form, miteinander oder aber in einer besonders bevorzugten Form mit Aroma- oder Geschmackstoffen kombiniert werden.

Als Aromastoffe eignen sich sowohl komplexe natürliche Rohstoffe wie aus Pflanzen gewonnene Extrakte und etherische Öle, bzw. daraus gewonnene Fraktionen und ein-heitliche Stoffe, als auch einheitliche synthetisch oder biotechnologisch gewonnene Aromastoffe.

### Beispiele für natürliche Rohstoffe sind z.B.:

Pfefferminzöle, Krauseminzöle, Mentha-Arvensis-Öle, Anisöle, Nelkenöle, Citrus-öle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Scharfgarbenöle, Sternanisöle, Thymianöle, Wacholderbeeröle, Rosmarinöle, Angelikawurzelöle, und die Fraktionen dieser Öle.

### Beispiele für einheitliche Aromastoffe sind z.B.:

Anethol, Menthol, Menthon, Isomenthon, Menthylacetat, Menthofuran, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvyl-acetat, p-Cymol, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 2-Phenylethylalkohol, 2-Phenylethylisabutyrat, 2-Phenylethylisovalerat, Zimtaldehyd, Geraniol, Nerol. Bei chiralen Verbindungen können die Aromastoffe als Racemat oder als einzelnes Enantiomer oder als enantiomerenangereichertes Gemisch vorliegen.

Beispiele für weitere Geschmarkstoffe, die vorteilhaft mit den erfindungsgemäß enthaltenen Rhinologica kombiniert werden können, sind z.B. Stoffe mit einer physiologischen Kühlwirkung, d.h. Stoffe, die in den Schleimhäuten eine Kälteempfindung hervorrufen. Solche Kühlwirkstoffe sind z.B. 1-Menthol, 1-Isopulegol, Menthonglycerinacetal, Menthyllactat, substituierte Menthan-3-carbonsäureamide (z.B. Menthan-3-carbonsäure-N-ethylamid), 2-Isopropyl-N,2,3-trimethylbutanamid, substituierte Cyclohexancarbonsäureamide, 3-Menthoxy-1,2-propandiol, 2-Hydroxyethylmenthylcarbonat, 2-Hydroxypropylmenthylcarbonat, N-Acetylglycinmenthylester, Methylhydroxycarbonsäureester (z.B. Menthyl-3-hydroxybutyrat), Menthylmonosuccinat, 2-Mercaptocyclodecanon, Menthyl-2-pyrrolidin 5-oncarboxylat.

Die erfindungsgemäß enthaltenen Rhinologica können in den Aroma-, bzw. GeschmadksstoYkompositionen zu einem Anteil von 0,1 bis 100 Gew.% vorliegen. Bevorzugt ist ein Gehalt von 0,5 bis 70 Gew.%; besonders bevorzugt ein Gehalt von 0,5 bis 40 Gew.%.

Die die Rhinologica enthaltenden Aroma- oder Geschmacksstoffkompositionen können in reiner Form, als Lösungen oder auch in besonders zu bereiteter Form verwendet und in gebrauchsfertige Produkte eingearbeitet werden.

Als Lösungsmittel eignen sich z.B. Ethylalkohol, 1,2-Propylenglycol, Triacetin, Benzylakohol und fette Öle wie z.B. Kokosöl oder Sonnenblumenöl.

Die die Rhinologica enthaltenden Aroma- oder Geschmacksstoffkompositionen können auch Zusatz- und Hilfsstoffe wie z.B. Konservierungsstoffe, Farbstoffe, Antioxidantien, Fließmittel, Verdickungsmittel, etc. enthalten.

In besonderen zubereiteteten Formen können die die Rhinologica enthaltenden Aroma- oder Geschmacksstosffkompositionen an einen Träger gebunden, sprühgetrocknet oder auch verkapselt vorliegen.

In der gebundenen Form können die Aroma- oder Geschmacksstoffkompositionen an oder in einen Träger gebunden sein, z.B. Kochsalz, Zucker, Stärken oder Zuckerschmelzen.

Die sprühgetrocknete Form wird aus den flüssigen Kompositionen hergestellt, indem man eine Emulsion unter Zugabe von bestimmten Mengen eines Trägerstoffs, vorzugsweise Biopolymere wie Stärke, modizierte Stärken, Maltodextrin und Gummi Arabicum, herstellt. Diese Emulsion wird in Sprühtrocknern durch Feinstverteilung bei gleichzeitiger Temperaturanwendung getrocknet. Es resultiert ein Pulver mit der gewünschten Beladung an flüssiger Komposition.

Die verkapselte Form wird ebenfalls aus den flüssigen Kompositionen durch Zugabe eines Trägerstoffs hergestellt. Es gibt verschiedenen Technologien, mit denen Kapseln hergestellt werden können. Die gängigsten sind die Extrusion, die Sprühgranulation und die Coazervation. Die Partikelgrößen reichen üblicherweise von 10 µm bis 5 mm. Die gängigsten Kapselmaterialien sind verschiedene Stärken, Maltodextrin und Gelatine. In diesen Kapseln sind die flüssigen oder festen Aroma- oder Geschmacksstoffkompositionen eingeschlossen und können durch verschiedene Mechanismen wie Wärmeanwendung, pH-Verschiebung oder Kaudruck freigesetzt werden.

Die erfindungsgemäß enthaltenen Rhinologica einen sich zur Herstellung von Zubereitungen der unterschiedlichsten Geschmacksrichtungen, besonders aber für die Verwendung in Aromakompositionen mit einem kühl-erfrischenden Minze-artigen Geschmack. Diese Minz-Kompositionen sind im wesentlichen gekennzeichnet durch einen Gehalt an Pfefferminzölen, Mentha-arvensis-Ölen, Krauseminzölen, Eucalyptusölen, 1,8-Cineol (Eucalyptol), Menthol und Substanzen mit physiologischer Kühlwirkung.

Die Gehalte der einzelnen Kompositionsbestandteile der Minz-Kompositionen können dabei im Allgemeinen zwischen 0,1 und 99,9 % variieren.

Bevorzugt verwendet werden Minz-Kompositionen mit 1 bis 90 Gew.-% Menthol, 1 bis 60 Gew.-% Menthon, 1 bis 90 Gew.-% Pfefferminz-, bzw. Mentha-arvensis-Ölen, 1 bis 90 Gew.-% Krauseminzölen, 1 bis 90 Gew.-% Eucalyptol bzw. Eucalyptolhaltigen Eucalyptusölen, 0,5 bis 70 Gew.% der erfindungsgemäßen Rhinologica, z.B. Menthylmethylether, Isopulegylmethylether o.ä. und 0,5 bis 70 Gew.-% Stoffen mit physiologischer Kühlwirkung.

Besonders bevorzugt verwendet werden Minz-Kompositionen mit 20 bis 60 Gew.-% Menthol, 5 bis 30 Gew.% Menthon, 5 bis 60 Gew.-% Pfefferminz-, bzw. Menthaarvensis-Ölen, 5 bis 60 Gew.-% Krauseminzölen, 2 bis 50 Gew.-% Bucalyptol bzw. Eucalyptol-haltigen Eucalyptusölen, 0,5 bis 40 Gew.-% der erfindungsgemäßen Rhinologica, z.B. Menthylmethylether, Isopulegylmethylether o.ä. und 1 bis 30 Gew.% Stoffen mit physiologischer Kühlwirkung.

Stoffe mit physiologischer Kühlwirkung können die oben beschriebenen sein, wobei sie einzeln oder als Gemische eingesetzt werden können. Wenn Gemische verwendet 1, werden, handelt es sich im Allgemeinen um Gemische z.B. aus Menthonglycerinacetal, Menthyllactat, substituierte Menthyl-3-carbonsäureamide (z.B. Menthyl-3-carbonsärue-N-ethylamid), 2-Hydroxyethylmenthylcarbonat und 2-Hydroxypropylmenthylcarbonat.

Im Allgemeinen werden Gemische mit 1 bis 99 Gew.-% Menthonglycerinacetal, 1 bis 99 Gew.-% Menthyllactat, 1 bis 99 Gew.-% Menthyl-3-carbonsäure-N-ethylamid, 1 bis 99 Gw.-% 2-Hydroxyethylmenthylcarbonat und 1 bis 99 Gew.-% Hydroxy-propylmenthylcarbonat verwendet.

Bevorzugt sind Gemische mit 1 bis 70 Gew.-% Menthonglycerinacetal, 1 bis 70 Gew.-% Menthyllactat, 1 bis 70 Gew.-% Menthyl-3-carbonsäure-N-ethylamid, 1 bis 70 Gew.-% 2-Hydroxyethylmenthylcarbonat und 1 bis 70 Gew.-% 2-Hydroxypropylmenthylcarbonat.

Durch Zusatz weiterer Aromastoffe, z.B. vom Typ süß, süß-aromatisch, frisch, fruchtig oder gegebenenfalls auch von weiteren Geschmacksrichtungen, können diese Minz-Kompositionen geschmacklich modifiziert werden, wobei der Gewichtsanteil der zugesetzten Aromastoffe im Allgemeinen 0,001 bis 50 Gew.-%, bezogen auf den Gewichtsanteil der Minz- und Kühlwirkstoffe, betragen kann. Bevorzugt ist ein Zusatz von 0,01 bis 30 Gew.-%; besonders bevorzugt ein Zusatz von 0,1 bis 10 Gew.-%, bezogen auf den Gewichtsanteil der Minz- und Kühlwirkstoffe.

Durch die Verwendung der erfindungsgemäß enthaltenen Rhinologica in derartigen Kompositionen kann der Anteil der Eucalyptusölen und des 1,8-Cineols (Eucalyptols) und damit die strenge medizinische Geschmacksnote reduziert werden, ohne dass das frische befreiende Gefühl im Mund, Rachenraum und in den Atemwegen vermindert wird. Das Frischeempfinden, das durch die erfindungsgemäß enthaltenen Rhinologica in den Atemwegen, im Mund und Rachenraum erzeugt wird, ist von längerer Zeitdauer als das von 1,8-Cineol (Eucalyptol) hervorgerufene.

Bemerkenwert ist dabei, dass die Geschmacksintensität und die Geschmacksfülle der Minz-Kompositionen durch den Einsatz der erfindungsgemäß enthaltenen Rhinologica erhöht und die kühlende Wirkung der Stoffe mit einer physiologischen Kühlwirkung verstärkt wird.

Die die Rhinologica enthaltenden Zubereitungen können vorteilhaft vor allem in Mundpflegemitteln, wie Zahnpasten und Mundwässern, Kaugummis, Nahrungsmitteln, wie Süßwaren und Lutschbonbons, Genußmitteln, wie Tabak, pharmazeutischen Präparaten, Nasensprays, eingesetzt werden.

Der Gehalt der die Rhinologica enthaltenden Zubereitungen beträgt in gebrauchsfertigen Mundwässem 0,01 bis 1 Gew.-%, besonders bevorzugt ist ein Gehalt von 0,1 bis 0,3 Gew.-%. In Mundwasserkonzentraten liegt der Gehalt der die Rhinologica enthaltenden Kompositionen zwischen 0,01 und 20 Gew.-%, bevorzugt ist ein Gehalt von 0,1 bis 10 Gew.-%. besonders bevorzugt ein Gehalt von 3 bis 5 Gew.-%. In Zahnpasten und Kaugummis werden die die Rhinologica enthaltenden Kompositionen in einer Konzentration zwischen 0,1 und 5 Ges.-% verwendet, bevorzugt ist ein Gehalt von 0,5 bis 2 Gew.-%. besonders bevorzugt ein Gehalt zwischen 0,8 und 1,5 Gew.-%. In Lutschbonbons beträgt der Gehalt der die Rhinologica enthaltenden Kompositionen zwischen 0,01 und 2 Gew.-%, bevorzugt ist ein Gehalt von 0,05 bis 1 Gew.-%; besonders bevorzugt ein Gehalt zwischen 0,1 und 0,5 %.

Zahnpasten, die mit den die Rhinologica enthaltenden Kompositionen aromatisiert werden, bestehen im allgemeinen aus einem abrasiven System (Schleif- oder Poliermittel), wie z.B. Kieselsäuren, Calciumcarbonaten, Calciumphosphaten, Alumiuniumoxiden und/oder Hydroxylapatiten, aus oberflächenaktiven Substanzen, wie z.B. Natriumlaurylsulfat, Natriumlaurylsarcosinat und/oder Cocamidopropylbetain, aus Feuchthaltemitteln, wie z.B. Glycerin und/oder Sorbit, aus Verdickungsmitteln, wie z.B. Carboxymethylcellulose, Polyethylenglycolen, Carra-geenanen und/oder Laponiten^{®}, aus Süßstoffen, wie z.B. Saccharin, aus Stabilisatoren und aus aktiven Wirkstoffen, wie z.B. Natriumfluorid, Natriummonofluorphos-phat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiurnchlorid, Wasserstoffperoxid, und/oder Natriumbicarbonat

Kaugummis, die mit den die Rhinologica enthaltenden Kompositionen aromatisiert werden, bestehen im allgemeinen aus einer Kaugummibase, d.h. einer beim Kauen plastische werdenden Kaumasse, aus Zuckern verschiedener Arten, Zuckeraustauschstoffen, Süßstoffen, Zuckeralkoholen, Feuchthaltemitteln, Verdickern, Emulgatoren und Stabilisatoren.

Bei der Anwendung von Fertigprodukten, die Kompositionen mit den Rhinologica enthalten, zeigt es sich, dass die erfindungsgemäßen die Rhinologica enthaltenden Kompositionen sich besonders dafür eignen, die Atemluft zu erfrischen und schlechten Mundgeruch zu neutralisieren, bzw. zu reduzieren.

Die Verwendung der erfindungsgemäß enthaltenen Rhinologica, bzw. der die Rhinologica enthaltenden Kompositionen in Mundpflegeprodukten, wie z. B. Mundwässern und Zahnpasten, und Kaugummis führt dazu, dass unangenehme, vor allem bittere Geschmackseindrücke maskiert oder neutralisiert werden, die z.B. durch Stoffe wie Triclosan, Zinkcitrat, -sulfat, Poly- und Pyrophosphaten, Bicarbonate, Strontium- und Kaliumsalze, Zinnpyrophosphat, -chlorid, Aluminiumlactat, Wasserstoffperoxid, Fluoride, Vitamine, Cetylpyridiniumchlorid sowie von Emulgatoren, wie z.B. besonders Natriumlaurylsulfat, Natriumlaurylsarkosinat und Cocamidopropylbetain, und Süßstoffen, wie z.B. Aspartam, Saccharin, Acesulfam-K, Sorbit; Xylit, Cyclamate (z.B. Natriumcyclamat), Sucralose, Alitam, Neotam, Thaumatin, Neohesperidin DC, Maltit, Lactit oder Kaugummi-Massen hervorgerufen werden.

Als weitere positive Eigenschaft der erfindungsgemäß enthaltenen Rhinologica ist ihre Stabilität in Zahnpasten auf Kreise- oder Bicarbonatbasis hervorzuheben, die wegen ihres alkalischen pH-Wertes schwierig zu aromatisieren sind.

Die erfindungsgemäß enthaltenen Rhinologica, bzw. die erfindungsgemäßen Rhinologica enthaltenden Kompositionen eignen sich jedoch auch für den Einsatz in pharmazeutischen Präparaten, wie z. B. Nasentropfen und -sprays oder Einreibepräparaten. Insbesondere eignen sich die die erfindungsgemäßen Rhinologica enthaltenden Kompositionen für die Maskierung des bitteren Geschmacks von Medikamenten.

### Beispiele:

Die nachfolgenden Beispiele sollen den Gebrauch der erfindungsgemäß enthaltenen Rhinologica verdeutlichen. Der Gebrauch der erfindungsgemäß enthaltenen Rhinologica ist jedoch nicht auf die angeführten Beispiele beschränkt.

### Beispiel 1: Herstellung der Alkylether

### Allgemeine Vorschrift:

1 mol des zu verethernden Alkohols wird in 400 ml Toluol gelöst und nach Zugabe von 2,6 mol 50-proz Natronlauge und 2g Tetrabutylammoniumiodid kräftig durchgerührt. Bei einer Sumpftemperatur von max. 45°C werden 1,2 mol Alkylierungsmittel während 1h dosiert. Anschließend wurde noch 3h bei dieser Temperatur gerührt. Falls Dialkylsulfate als Alkylierungsmittel verwendet wurden, wird zu ihrer Zerstörung mit Ammoniak versetzt und noch 30 min bei Raumtemperatur gerührt. Nach Zugabe von Wasser werden die Phasen getrennt. Von der organischen Phase wird das Lösungsmittel abgezogen und der Rückstand destillativ aufgetrennt. Die reinen Ether werden durch Redestillation über eine Spaltrohrkolonne erhalten.

Auf diese Weise wurden folgende Ether hergestellt:

| Verbindung: | | | | | | Massenspektrum: | | | | | | |
|---|---|---|---|---|---|---|---|---|---|---|---|---|
| Menthylmethylether | | | | | | | | | | | | |
| m/z = | 170 | 155 | 138 | 123 | 95 | 85 | 81 | 67 | 55 | 45 | 41 | |
| % | 1 | 2 | 45 | 19 | 36 | 100 | 49 | 18 | 27 | 15 | 28 | |

| Menthylethylether | | | | | | | | | | | | |
|---|---|---|---|---|---|---|---|---|---|---|---|---|
| m/z = | 184 | 138 | 123 | 113 | 99 | 95 | 81 | 71 | 55 | 41 | | |
| % | 2 | 36 | 12 | 8 | 100 | 20 | 26 | 28 | 18 | 20 | | |

| Menthylpropylether | | | | | | | | | | | | |
|---|---|---|---|---|---|---|---|---|---|---|---|---|
| m/z | 198 | 138 | 123 | 113 | 95 | 81 | 71 | 55 | 41 | | | |
| % | 2 | 46 | 16 | 100 | 26 | 33 | 63 | 22 | 29 | | | |

| Menthylisobutylether | | | | | | | | | | | | |
|---|---|---|---|---|---|---|---|---|---|---|---|---|
| m/z | 212 | 138 | 127 | 123 | 95 | 83 | 81 | 71 | 57 | 55 | 41 | |
| % | 2 | 43 | 76 | 21 | 34 | 38 | 53 | 100 | 43 | 28 | 34 | |

| 2-Isopropylcyclohexylmethylether | | | | | | | | | | | | |
|---|---|---|---|---|---|---|---|---|---|---|---|---|
| m/z | 156 | 141 | 124 | 113 | 109 | 99 | 95 | 81 | 71 | 67 | 55 | 41 |
| % | 5 | 12 | 100 | 16 | 58 | 21 | 23 | 60 | 90 | 36 | 25 | 56 |

| 2-Isopropylcyclohexylethylether | | | | | | | | | | | | |
|---|---|---|---|---|---|---|---|---|---|---|---|---|
| m/z | 170 | 155 | 127 | 124 | 109 | 99 | 95 | 85 | 81 | 67 | 57 | 41 |
| % | 8 | 16 | 24 | 100 | 55 | 18 | 20 | 96 | 50 | 26 | 75 | 38 |

| 1-(3,3-Dimethylcyclohexyl)ethylethylether | | | | | | | | | | | | |
|---|---|---|---|---|---|---|---|---|---|---|---|---|
| m/z | 184 | 123 | 81 | 73 | 69 | 55 | 45 | 41 | 28 | | | |
| % | 2 | 16 | 8 | 100 | 7 | 10 | 59 | 14 | 10 | | | |

| 1-(3,3-Dimethylcyclohexyl)ethylpropylether | | | | | | | | | | | | |
|---|---|---|---|---|---|---|---|---|---|---|---|---|
| m/z 198 183 123 87 | | | | | 81 | 69 | 55 | 45 | 41 | | | |
| % | 1 | 2 | 16 | 93 | 9 | 14 | 13 | 100 | 23 | | | |

| 1-(3,3-Dimethylcyclohexyl)ethylmethylether | | | | | | | | | | | | |
|---|---|---|---|---|---|---|---|---|---|---|---|---|
| m/z | 170 | 155 | 123 | 110 | 95 | 81 | 69 | 59 | 55 | 41 | | |
| % | 1 | 3 | 13 | 5 | 6 | 9 | 7 | 100 | 10 | 12 | | |

| 3,3,5-Trimethylcyclohexylmethylether | | | | | | | | | | | | |
|---|---|---|---|---|---|---|---|---|---|---|---|---|
| m/z | 156 | 141 | 124 | 109 | 99 | 85 | 67 | 58 | 55 | 41 | | |
| % | 4 | 3 | 29 | 61 | 87 | 100 | 23 | 18 | 28 | 30 | | |

| Isopulegylmethylether | | | | | | | | | | | | |
|---|---|---|---|---|---|---|---|---|---|---|---|---|
| m/z | 168 | 153 | 136 | 121 | 111 | 98 | 93 | 85 | 81 | 67 | 55 | 41 |
| % | 18 | 13 | 9 | 41 | 30 | 34 | 28 | 100 | 24 | 21 | 23 | 25 |

### Beispiel 2

Herstellung eines Zahnpasta-Aromas vom Eucalyptus-Menthol-Typ unter Verwendung von Menthylmethylether:

Es wurden vermischt:

| | | |
|---|---|---|
| 0,5 | Gew.-Tle. | Campher |
| 3 | Gew.-Tle. | Anethol |
| 6 | Gew.-Tle. | Pfefferminzöl vom Typ Mentha arvensis |
| 2 | Gew.-Tle. | Menthyllactat |
| 2 | Gew.-Tle. | 2-Hydroxyethylinenthylcarbonat |
| 2 | Gew.-Tle. | 2-Hydroxypropylmenthyl carbonat |
| 20 | Gew.-Tle. | 1,8-Cineol (Eucalyptol) |
| 64,5 | Gew.-Tle. | /-Menthol |

In einem zweiten Mischansatz wurde der Anteil des 1,8-Cineols (Eucalyptols) durch *l*-Menthylmethylether ersetzt. Beide Aromen wurden in eine Standard-Zahnpasta-Masse auf Kieselsäure-Basis in einer Konzentration von 1,3 Gew.% eingearbeitet. Beide Zahnpasten wurden von einem sensorisch geschulten Expertenpanel unter Praxisbedingungen getestet. Das Ergebnis zeigte, dass durch das Aroma mit /Menthylmethylether und ohne 1,8-Cineol (Eucalyptol) der starke medizinische Geschmack deutlich herabgesetzt wurde, ohne dass der Geschmackseindruck von Frische in den Atemwegen, im Mund und Rachenraum reduziert wurde. Insgesamt führte das zweite Aroma zu einem klareren, längeranhaltenden Geschmackseindruck mit mehr Fülle und Volumen.

Vergleichbare Effekte werden erhalten, wenn an Stelle des *l*-Menthylmethylethers Isopulegylmethylether, 3,3,5-Trimethylcyclohexylmethylether oder 1-(3,3-Dimethylcyclohexyl)ethylmethylether verwendet werden.

### Beispiel 3

Herstellung eines Zahnpasta-Aromas vom Wintergrün-Typ unter Verwendung von Menthylmethylether:

Es wurden vermischt:

| | | |
|---|---|---|
| 10 | Gew.-Tle. | Anethol |
| 5 | Gew.-Tle. | Pfefferminzöl vom Typ Mentha arvensis |
| 5 | Gew.-Tle. | Pfefferminzöl vom Typ Mentha piperita |
| 25 | Gew.-Tle. | Methylsalicylat |
| 40 | Gew.-Tle. | *l*-Menthol |
| 15 | Gew.-Tle. | *l*-Menthylmethylether |

Das Aroma wurde in eine Standard-Zahnpasta-Masse auf Kieselsäure-Basis in einer Konzentration von 1,3 Gew.% eingearbeitet. Die Zahnpasta wurde von einem sensorisch geschulten Expertenpanel unter Praxisbedingungen getestet. Das Ergebnis zeigte, dass sich durch das Aroma ein Geschmackseindruck von hoher Intensität und lang anhaltender Dauer ergab, wobei ein ausgeprägtes, deutlich frisches, befreiendes Gefühl in den Atemwegen, im Mund und im Rachenraum wahrgenommen wurde.

### Beispiel 4

Herstellung eines Kaugummi-Aromas unter Verwendung von *l*-Menthylmethylether:
Es wurden vermischt:

| | | |
|---|---|---|
| 40 | Gew.-Tle. | Pfefferminzöl vom Typ Mentha arvensis |
| 20 | Gew.-Tle. | *l*-Menthon |
| 20 | Gew.-Tle. | /-Menthol |
| 20 | Gew.-Tle. | *l*-Menthyhmethylether |

Das Aroma wurde in eine zuckerfreie Standard-Kaugummi-Masse in einer Konzentration von 1,5 Gew.% eingearbeitet. Das Kaugummi wurde von einem geschulten Expertenpanel auf seine sensorische Qualität getestet. Es zeigte sich, dass durch die Zugabe des *l*-Menthylmethylethers ein deutlich frisches befreiendes Gefühl in den Atemwegen, im Mund und im Rachenraum erhalten wurde und die Geschmacksfülle und Intensität des Pfefferminzaromas deutlich erhöht wurde.

## Patentansprüche

1. Zubereitungen mit Frischewirkung, enthaltend Verbindungen der Formel (I), 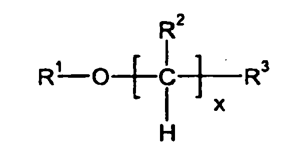 worin
x den Wert 0 oder 1 annehmen kann,
R¹ eine Alkylgruppe mit 1 bis 4 C-Atomen.
R² eine Methyl- oder Ethylgruppe und
R³ ein monocyclisches Kohlenstoffsystem mit 5, 6, 7 oder 8 Kohlenstoffatomen, das gegebenenfalls mit weiteren Alkylgruppe mit 1 bis 4 C-Atomen oder Alkenylgruppen mit 2 bis 4 C-Atomen substituiert sein kann,
und die ausgewählt sind aus der Gruppe bestehend aus I-Menthylmethylether, d-Menthylmethylether, dl-Menthylmethylether, Menthylethylether, Menthylpropylether, Menthylisobutylether, Isopulegylmethylether, 2-Isopropylcyclohexylmethylether, 2-Isopropylcyclohexylethylether, 3,3,5-Trimethylcyclohexylmethylether, 1-(3,3-Dimethylcyclohexyl)ethylethylether, 1-(3,3-Dimethylcyclohexyl)ethylpropylether, 1-(3,3-Dimethylcyclohexyl)ethylmethylether, **dadurch gekennzeichnet, dass** die Zubereitungen 0,0001 bis 10 Gew.% der Verbindung der Formel (I) enthalten.

2. Verwendung von Zubereitungen nach Anspruch 1 in Mundpflegemitteln.

3. Verwendung nach Anspruch 2, **dadurch gekennzeichnet, dass** die Mundpflegemittel Zahnpasten sind.

4. Verwendung nach Anspruch 2, **dadurch gekennzeichnet, dass** die Mundpflegemittel Mundwässer sind.

5. Verwendung nach Anspruch 2, **dadurch gekennzeichnet, dass** die Mundpflegemittel Kaugummi sind.

6. Verwendung von Zubereitungen nach Anspruch 1 in Nahrungsmitteln.

7. Verwendung von Zubereitungen nach Anspruch 1 in Genussmitteln.

8. Verwendung nach Anspruch 6, **dadurch gekennzeichnet, dass** die Nahrungsmittel Süßwaren sind.

9. Verwendung von Zubereitungen nach Anspruch 1 in pharmazeutischen Präparaten.

10. Verwendung einer Verbindung der Formel (I) 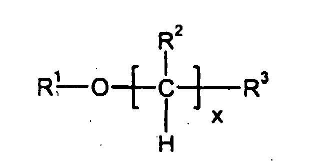 worin
x den Wert 0 oder 1 annehmen kann,
R¹ eine Alkylgruppe mit 1 bis 4 C-Atomen,
R² eine Methyl- oder Ethylgruppe und
R³ ein monocyclisches Kohlenstoffsystem mit 5, 6, 7 oder 8 Kohlenstoffatomen, das gegebenenfalls mit weiteren Alkylgruppe mit 1 bis 4 C-Atomen oder Alkenylgruppen mit 2 bis 4 C-Atomen substituiert sein kann,
zum Hervorrufen eines frischen und befreienden Gefühls im Bereich des Mundes, des Rachens und der Atemwege.

11. Verwendung nach Anspruch 10, wobei die Verbindung der Formel (I) ausgewählt ist aus der Gruppe bestehend aus I-Menthylmethylether, d-Menthylmethylether, dl-Menthylmethylether, Menthylethylether, Menthylpropylether, Menthylisobutylether, Isopulegylmethylether, 2-Isopropylcyclohexylmethylether, 2-Isopropylcyclohexylethylether, 3,3,5-Trimethylcyclohexylmethylether, 1-(3,3-Dimethylcyclohexyl)ethylethylether, 1-(3,3-Dimethylcyclohexyl)ethylpropylether, 1-(3,3-Dimethylcyclohexyl)ethylmethylether.

12. Rhinologica der Formel (I), 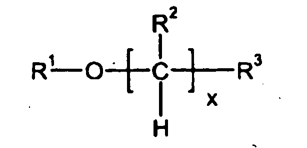 worin
x den Wert 0 oder 1 annehmen kann,
R¹ eine Alkylgruppe mit 1 bis 4C-Atomen,
R² eine Methyl- oder Ethylgruppe und
R³ ein monocyclisches Kohlenstoffsystem mit 5, 6, 7 oder 8 Kohlenstoff-atomen, das gegebenenfalls mit weiteren Alkylgruppe mit 1 bis 4 C-Atomen oder Alkenylgruppen mit 2 bis 4 C-Atomen substituiert sein kann,
und die ausgewählt sind aus der Gruppe bestehend aus, Isopulegylmethylether, 2-Isopropylcyclohexylethylether, 3,3,5-Trimethylcyclohexylmethylether, 1-(3,3-Dimethylcyclohexyl)ethylethylether, 1-(3,3-Dimethylcyclohexyl)ethylpropylether, 1-(3,3-Dimethylcyclohexyl)ethylmethylether.

13. Aroma- oder Geschmacksstoffmischung mit Frischewirkung, enthaltend Verbindungen der Formel (I), 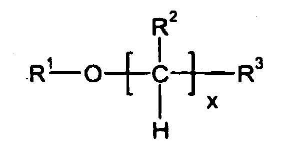 worin
x den Wert 0 oder 1 annehmen kann,
R¹ eine Alkylgruppe mit 1 bis 4 C-Atomen,
R² eine Methyl- oder Ethylgruppe und
R³ ein monocyclisches Kohlenstoffsystem mit 5, 6, 7 oder 8 Kohlenstoff-atomen, das gegebenenfalls mit weiteren Alkylgruppe mit 1 bis 4 C-Atomen oder Alkenylgruppen mit 2 bis 4 C-Atomen substituiert sein kann,
und die ausgewählt sind aus der Gruppe bestehend aus I-Menthylmethylether, d-Menthylmethylether, dl-Menthylmethylether, Menthylethylether, Menthylpropylether, Menthylisobutylether, Isopulegylmethylether, 2-Isopropylcyclohexylmethylether, 2-Isopropylcyclohexylethylether, 3,3,5-Trimethylcyclohexylmethylether, 1-(3.,3-Dimethylcyclohexyl)ethylethylether, 1-(3,3-Dimethylcyclohexyl)ethylpropylether, 1-(3,3-Dimethylcyclohexyl)ethylmethylether,
sowie wenigstens einen weiteren Aroma- oder Geschmacksstoff.

## Claims

1. Preparations having a freshening effect, containing compounds of formula (I), 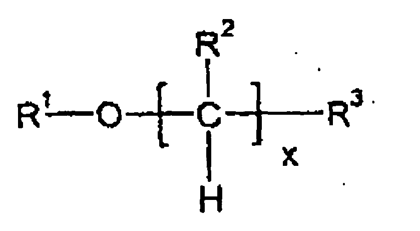 where
x can take the value 0 or 1,
R¹ is an alkyl group having from 1 to 4 C atoms,
R² is a methyl or ethyl group and
R³ is a monocyclic carbon system having 5, 6, 7 or 8 carbon atoms, which may optionally be substituted with another alkyl group having from 1 to 4 C atoms or further alkenyl groups having from 2 to 4 C atoms,
and which are selected from the group consisting of 1-menthyl methyl ether, d-menthyl methyl ether, dl-menthyl methyl ether, menthyl ethyl ether, menthyl propyl ether, menthyl isobutyl ether, isopulegyl methyl ether, 2-isopropyl-cyclohexyl methyl ether, 2-isopropyl-cyclohexyl ethyl ether, 3,3,5-trimethyl-cyclohexyl methyl ether, 1-(3,3-dimethyl-cyclohexyl) ethyl ethyl ether, 1-(3,3-dimethyl-cyclohexyl) ethyl propyl ether, 1-(3,3-dimethyl-cyclohexyl) ethyl methyl ether, **characterised in that** the preparations contain from 0.0001 to 10% by weight of the compound of formula (I).

2. Use of preparations according to Claim 1 in oral hygiene products.

3. Use according to Claim 2, **characterised in that** the oral hygiene products are toothpastes.

4. Use according to Claim 2, **characterised in that** the oral hygiene products are mouthwashes.

5. Use according to Claim 2, **characterised in that** the oral hygiene products are chewing gum.

6. Use of preparations according to Claim 1 in food products.

7. Use of preparations according to Claim 1 in drink and tobacco products.

8. Use according to Claim 6, **characterised in that** the food products are confectionery.

9. Use of preparations according to Claim 1 in pharmaceutical preparations.

10. Use of a compound of formula (I) 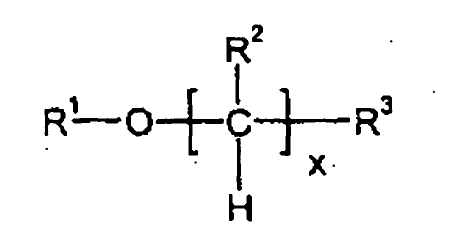 where
x can take the value 0 or 1,
R¹ is an alkyl group having from 1 to 4 C atoms,
R² is a methyl or ethyl group and
R³ is a monocyclic carbon system having 5, 6, 7 or 8 carbon atoms, which may optionally be substituted with another alkyl group having from 1 to 4 C atoms or further alkenyl groups having from 2 to 4 C atoms,
to produce a fresh and liberating feeling in the region of the mouth, the throat and the airways.

11. Use according to Claim 10, wherein the compound of formula (I) is selected from the group consisting of 1-menthyl methyl ether, d-menthyl methyl ether, dl-menthyl methyl ether, menthyl ethyl ether, menthyl propyl ether, menthyl isobutyl ether, isopulegyl methyl ether, 2-isopropyl-cyclohexyl methyl ether, 2-isopropyl-cyclohexyl ethyl ether, 3,3,5-trimethyl-cyclohexyl methyl ether, 1-(3,3-dimethylcyclohexyl) ethyl ethyl ether, 1-(3,3-dimethyl-cyclohexyl) ethyl propyl ether, 1-(3,3-dimethyl-cyclohexyl) ethyl methyl ether.

12. Rhinological agents of formula (I), 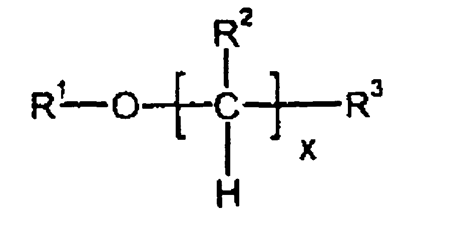 where
x can take the value 0 or 1,
R¹ is an alkyl group having from 1 to 4 C atoms,
R² is a methyl or ethyl group and
R³ is a monocyclic carbon system having 5, 6, 7 or 8 carbon atoms, which may optionally be substituted with another alkyl group having from 1 to 4 C atoms or further alkenyl groups having from 2 to 4 C atoms,
and which are selected from the group consisting of isopulegyl methyl ether, 2-isopropyl-cyclohexyl ethyl ether, 3,3,5-trimethyl-cyclohexyl methyl ether, 1-(3,3-dimethyl-cyclohexyl) ethyl ethyl ether, 1-(3,3-dimethyl-cyclohexyl) ethyl propyl ether, 1-(3,3-dimethyl-cyclohexyl) ethyl methyl ether.

13. Aromatising or flavouring agent mixture having a freshening effect, containing compounds of formula (I), 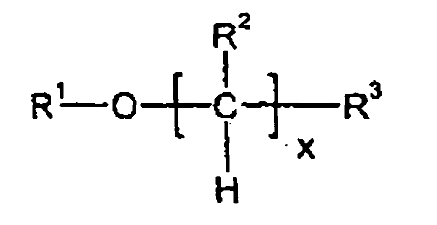 where
x can take the value 0 or 1,
R¹ is an alkyl group having from 1 to 4 C atoms,
R² is a methyl or ethyl group and
R³ is a monocyclic carbon system having 5, 6, 7 or 8 carbon atoms, which may optionally be substituted with another alkyl group having from 1 to 4 C atoms or further alkenyl groups having from 2 to 4 C atoms,
and which are selected from the group consisting of 1-menthyl methyl ether, d-menthyl methyl ether, dl-menthyl methyl ether, menthyl ethyl ether, menthyl propyl ether, menthyl isobutyl ether, isopulegyl methyl ether, 2-isopropyl-cyclohexyl methyl ether, 2-isopropyl-cyclohexyl ethyl ether, 3,3,5-trimethyl-cyclohexyl methyl ether, 1-(3,3-dimethyl-cyclohexyl) ethyl ethyl ether, 1-(3,3-dimethyl-cyclohexyl) ethyl propyl ether, 1-(3,3-dimethyl-cyclohexyl) ethyl methyl ether,
and at least one further aromatising or flavouring agent.

## Revendications

1. Préparations avec une action rafraîchissante contenant des composés de la formule (I), 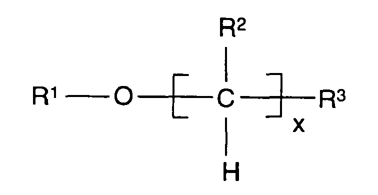 dans laquelle
x peut prendre la valeur 0 ou 1,
R¹ est un groupe alkyle avec de 1 à 4 atomes C,
R² est un groupe méthyle ou éthyle et
R³ est un système hydrocarboné monocyclique avec 5, 6, 7 ou 8 atomes de carbone, lequel peut éventuellement être substitué avec d'autres groupes alkyle avec de 1 à 4 atomes C ou groupes alcényle avec de 2 à 4 atomes C,
et lesquels sont choisis parmi le I-menthylméthyléther, le d-menthylméthyléther, le dl-menthylméthyléther, le menthyléthyléther, le menthyl-propyléther, le menthylisobutyléther, l'isopulégylméthyléther, le 2-iso-propylcyclohexylméthyléther, le 2-isopropylcyclohexyléthyléther, le 3,3,5-triméthylcyclohexylméthyléther, le 1-(3,3-diméthylcyclohexyl)éthyléthyl-éther, le 1-(3,3-diméthylcyclohexyl)éthylpropyléther, le 1-(3,3-diméthyl-cyclohexyl)éthylméthyléther, **caractérisés en ce que** les préparations contiennent de 0,0001 à 10 % en poids du composé de la formule (I).

2. Utilisation de préparations selon la revendication 1 dans des agents pour le soin buccal.

3. Utilisation selon la revendication 2, **caractérisée en ce que** les agents pour le soin buccal sont des dentifrices.

4. Utilisation selon la revendication 2, **caractérisée en ce que** les agents pour le soin buccal sont des eaux de bouche.

5. Utilisation selon la revendication 2, **caractérisée en ce que** les agents pour le soin buccal sont des pâtes à mâcher.

6. Utilisation des préparations selon la revendication 1 dans des aliments.

7. Utilisation des préparations selon la revendication 1 dans des denrées de luxe.

8. Utilisation selon la revendication 6, **caractérisée en ce que** les aliments sont des sucreries.

9. Utilisation des préparations selon la revendication 1 dans des préparations pharmaceutiques.

10. Utilisation d'un composé de la formule (I) 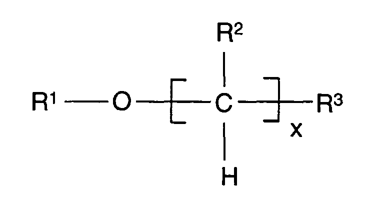 dans laquelle
x peut prendre la valeur 0 ou 1,
R¹ est un groupe alkyle avec de 1 à 4 atomes C,
R² est un groupe méthyle ou éthyle et
R³ est un système hydrocarboné monocyclique avec 5, 6, 7 ou 8 atomes de carbone, lequel peut éventuellement être substitué avec d'autres groupes alkyle ayant de 1 à 4 atomes C ou groupes alcényle ayant de 2 à 4 atomes C,
pour la fourniture d'une sensation fraîche et de dégagement dans le domaine de la bouche, de la trachée ou des voies respiratoires.

11. Utilisation selon la revendication 10, le composé de la formule (I) étant choisi parmi le I-menthylméthyléther, le d-menthylméthyléther, le dl-menthylméthyléther, le menthyléthyléther, le menthyl-propyléther, le menthylisobutyléther, l'isopulégylméthyléther, le 2-iso-propylcyclohexylméthyléther, le 2-isopropylcyclohexyléthyléther, le 3,3,5-triméthylcyclohexylméthyléther, le 1-(3,3-diméthylcyclohexyl)éthyléthyl-éther, le 1-(3,3-diméthylcyclohexyl)éthylpropyléther, le 1-(3,3-diméthyl-cyclohexyl)éthylméthyléther.

12. Produits de rhinilogie de la formule (I), 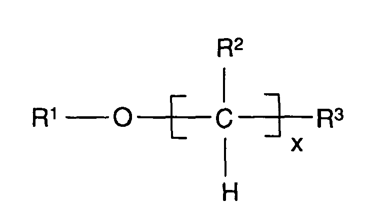 dans laquelle
x peut prendre la valeur 0 ou 1,
R¹ est un groupe alkyle avec de 1 à 4 atomes C,
R² est un groupe méthyle ou éthyle et
R³ est un système hydrocarboné monocyclique avec 5, 6, 7 ou 8 atomes de carbone, lequel peut éventuellement être substitué avec d'autres groupes alkyle ayant de 1 à 4 atomes C ou groupes alcényle ayant de 2 à 4 atomes C,
et lesquels sont choisis parmi l'isopulégylméthyléther, le 2-isopropylcyclohexyléthyléther, le 3,3,5-triméthylcyclohexylméthyléther, le 1-(3,3-diméthylcyclohexyl)éthyléthyléther, le 1-(3,3-diméthylcyclohexyl)éthyl-propyléther, le 1-(3,3-diméthylcyclohexyl)éthylméthyléther.

13. Mélange de matières d'arômes ou odorantes avec une action rafraîchissante contenant des composés de la formule (I), 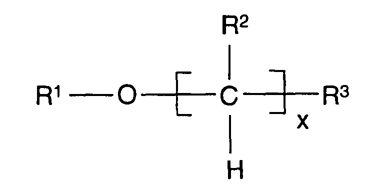 dans laquelle
x peut prendre la valeur 0 ou 1,
R¹ est un groupe alkyle avec de 1 à 4 atomes C,
R² est un groupe méthyle ou éthyle et
R³ est un système hydrocarboné monocyclique avec 5, 6, 7 ou 8 atomes de carbone, lequel peut éventuellement être substitué avec d'autres groupes alkyle ayant de 1 à 4 atomes C ou groupes alcényle ayant de 2 à 4 atomes C,
et lesquels sont choisis parmi le I-menthylméthyléther, le d-menthylméthyléther, le dl-menthylméthyléther, le menthyléthyléther, le menthyl-propyléther, le menthylisobutyléther, l'isopulégylméthyléther, le 2-iso-propylcyclohexylméthyléther, le 2-isopropylcyclohexyléthyléther, le 3,3,5-triméthylcyclohexylméthyléther, le 1-(3,3-diméthylcyclohexyl)éthyléthyl-éther, le 1-(3,3-diméthylcyclohexyl)éthylpropyléther, le 1-(3,3-diméthyl-cyclohexyl)éthylméthyléther,
ainsi qu'au moins une autre matière d'arôme ou odorante.
